Europäisches Patentamt

European Patent Office

Office européen des brevets

(19)

(11) **EP 1 148 907 B1**

(12) **EUROPÄISCHE PATENTSCHRIFT**

(45) Veröffentlichungstag und Bekanntmachung des Hinweises auf die Patenterteilung:
**10.12.2003 Patentblatt 2003/50**

(21) Anmeldenummer: **00903652.6**

(22) Anmeldetag: **28.01.2000**

(51) Int Cl.$^7$: **A61M 16/00**

(86) Internationale Anmeldenummer:
**PCT/EP00/00685**

(87) Internationale Veröffentlichungsnummer:
**WO 00/044427 (03.08.2000 Gazette 2000/31)**

(54) **NICHTINVASIVES VERFAHREN ZUR OPTIMIERUNG DER BEATMUNG ATELEKTATISCHER LUNGEN**

NON-INVASIVE METHOD FOR OPTIMIZING THE RESPIRATION OF ATELECTATIC LUNGS

PROCEDE NON INVASIF POUR L'OPTIMISATION DE LA RESPIRATION DE POUMONS ATELECTASIQUES

(84) Benannte Vertragsstaaten:
**AT BE CH CY DE DK ES FI FR GB GR IE IT LI LU MC NL PT SE**

(30) Priorität: **29.01.1999 DE 19903584**
**29.01.1999 DE 19903618**

(43) Veröffentlichungstag der Anmeldung:
**31.10.2001 Patentblatt 2001/44**

(73) Patentinhaber: **Siemens-Elema AB**
**171 95 Solna (SE)**

(72) Erfinder:
• **Böhm, Stephan, Dr.**
**20251 Hamburg (DE)**

• **Leonhardt, Steffen, Dr.**
**23566 Lübeck (DE)**

(74) Vertreter: **Joppich, Martin, Dr.-Ing. et al**
**Intellectual Property Consultancy**
**Zeppelinstrasse 71-73**
**81669 München (DE)**

(56) Entgegenhaltungen:
EP-A- 0 728 493     US-A- 5 103 814
US-A- 5 388 575     US-A- 5 660 170
US-A- 5 738 090     US-A- 5 752 509

# EP 1 148 907 B1

**Beschreibung**

Die Erfindung betrifft eine Vorrichtung zur Bestimmung des alveolären Öffnens und/oder des alveolären Schließens der Lunge.

Eine derartige Vorrichtung wird insbesondere zur optimalen Einstellung eines Beatmungsgerätes benötigt, da das alveoläre Öffnen und/oder alveoläre Schließen der Lunge wichtige physiologische Kenngrößen bei Patienten mit atelektatischen (= teilweise kollabierten) Lungen sind.

Auf den Intensivstationen in der Bundesrepublik Deutschland werden pro Tag etwa 8.000 - 10.000 Patienten beatmet. Die am Beatmungsgerät einzustellenden Größen, z.B. der Beatmungsdruck und die Atemfrequenz, werden meistens nach bewährten Standards ausgewählt, dann aber konstant gelassen und zu selten an die sich wandelnden Bedürfnisse der Patienten angepasst.

Zur Beurteilung des Beatmungserfolges wird in der Regel die arterielle Blutgasanalyse verwendet, bei der die Partialdrücke von Sauerstoff und Kohlendioxid ($paO_2$ und $paCO_2$) im Blut bestimmt werden. Allerdings werden diese Werte oft nur 1-4 mal pro Tag bestimmt. Wenn man bedenkt, dass ein Mensch pro Tag mehr als 20.000 Atemzüge ausführt, wird klar, dass diese "Abtastfrequenz" für eine wirkliche Beurteilung des Gasaustausches gerade bei kritischen und instabilen Patienten viel zu gering ist.

Zu dieser Gruppe gehören Patienten mit einem akuten Lungenversagen (ARDS = acute respiratory distress syndrome), einer Krankheit, die auch heute noch eine Sterblichkeit von 50% besitzt. Aufgrund eines Mangels an oberflächenaktiven Substanzen (sogenannter surfactant) kollabieren bei diesen Patienten große Teile der Lunge (sogenannte Atelektase) und stehen daher für den Gasaustausch nicht mehr zur Verfügung.

Ein erklärtes Therapieziel muss es sein, diese kollabierten Lungenareale möglichst frühzeitig und gleichzeitig schonend wieder zu "öffnen", um Folgeerkrankungen und konsekutives Organversagen zu vermeiden. Die Wahl der Beatmungsdrücke hat hierauf einen großen Einfluss.

Durch die Identifikation des alveolären Öffnens und insbesondere des alveolären Schließens der Lunge kann auch die kranke Lunge durch entsprechende Wahl der Beatmungsdrücke offen gehalten werden. Die manuelle Ermittlung der individuell verschiedenen Öffnungs- und Schließdrücke ist allerdings mühsam und zeitaufwendig. Um das Konzept auch für den klinischen Alltag praktikabel zu machen, empfiehlt es sich, diese Beatmungsstrategie mit Hilfe eines Computers zu automatisieren.

Bevor auf bekannte Maßnahmen zur automatischen Identifikation des Kollapspunktes der Lunge eingegangen wird, seien zunächst die grundsätzlichen Formen der künstlichen Beatmung beschrieben.

Die Hauptaufgabe der Lunge ist der Gasaustausch, d.h. die Versorgung des Körpers mit Sauerstoff und der Abtransport von Kohlendioxid. Für den Fall, dass ein Mensch den Gasaustausch nicht mehr selbstständig bewerkstelligen kann, muss er künstlich beatmet werden.

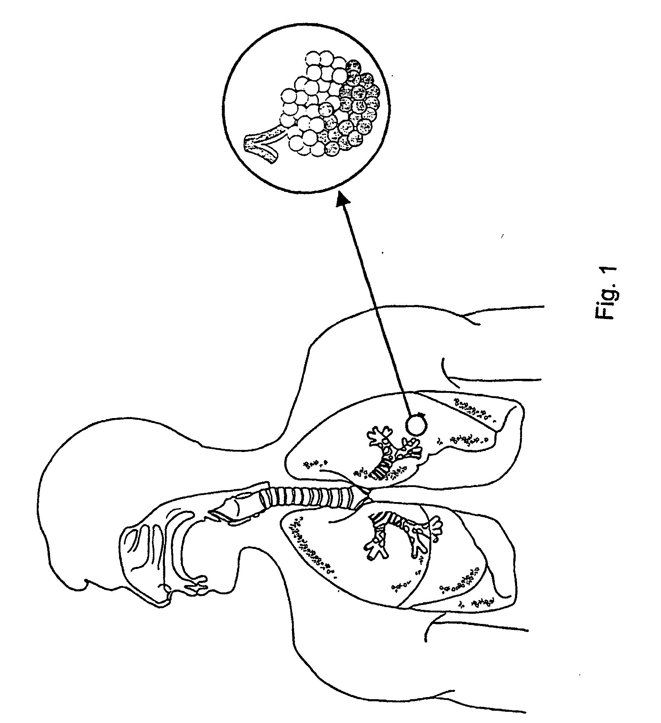

Fig. 1 zeigt den Bronchialbaum des Menschen und eine Ausschnittsvergrößerung von Alveolen der Lunge. Genau wie bei der Spontanatmung muss während der künstlichen Beatmung Frischluft aus der Umgebung in die Lungenbläschen gebracht werden. Bei diesen sogenannten "Alveolen" handelt es sich um sackförmige Ausstülpungen mit einem mittleren Durchmesser von ca. 70 $\mu$m, über deren Gesamtoberfläche der eigentliche Gasaustausch stattfindet.

Beim spontanatmenden Menschen wird in den Alveolen durch Anspannung der Atemmuskulatur ein Unterdruck erzeugt und dadurch Luft angesaugt. Bei der heute üblichen Form der künstlichen Beatmung wird hingegen am Mund des Patienten ein Überdruck appliziert, durch den Luft in die Lunge gedrückt wird (sogenannte "Überdruckbeatmung").

Generell wird zwischen unterstützender und kontrollierter Beatmung unterschieden. Bei der unterstützenden (assistierten) Beatmung wir die Atemaktivität des Patienten registriert. Diese Form der Beatmung wird z.B. in der Schlussphase einer Beatmung kurz vor dem Übergang zur Spontanatmung eingesetzt. Hingegen wird die kontrollierte Beatmung eingesetzt, wenn eine vollständige Kontrolle über die Atmung des Patienten erwünscht oder nötig ist. Bei der kontrollierten Beatmung unterscheidet man zwei Formen, die druckkontrollierte und die volumenkontrollierte Beatmung.

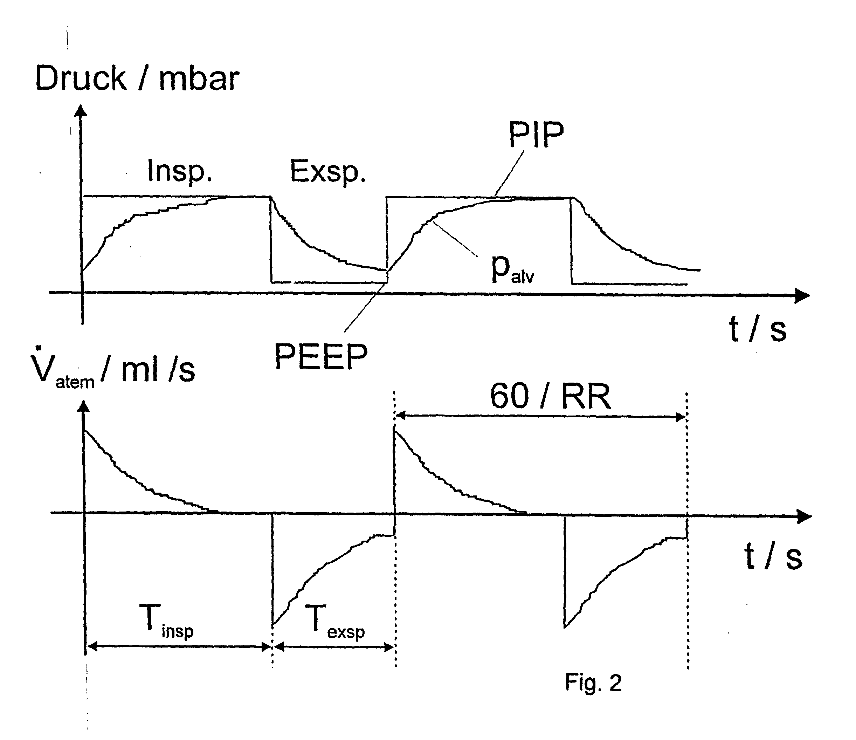

Bei der druckkontrollierten Beatmung wird der Beatmungsdruck sowohl während der Einatmungsphase (Inspiration) als auch während der Ausatmungsphase (Exspiration) vorgegeben. Die zugehörigen Drücke nennt man PIP (peak inspiratory pressure) und PEEP (positive endexspiratory pressure). Der Alveolardruck $P_{alv}$ pendelt entsprechend zwischen diesen beiden Druckwerten. Fig. 2 zeigt zur Veranschaulichung den Verlauf des Beatmungsdruckes und des alveolen Druckes sowie den Verlauf des Atemvolumenstroms bei der druckkontrollierten Beatmung.

Weitere frei wählbare Größen sind die Beatmungsfrequenz RR (respiratory rate) und das Ein- zu Ausatmungsverhältnis (I/E = inspriation to exspiration ratio). Hierbei gilt

$$RR = \frac{1}{T_{insp} + T_{exsp}} \cdot \frac{60}{min} \tag{1}$$

und

$$I/E = \frac{I_{insp}}{T_{exsp}} \tag{2}$$

mit $T_{insp}$ der Inspirationsdauer und $T_{exsp}$ der Exspirationsdauer. Das während eines Atemzuges bewegte Luftvolumen wird Tidalvolumen $V_T$ genannt. Unter der Voraussetzung eines Leckage-freien Systems und des stationären Falls gilt

$$V_T = \int_0^{T_{insp}} \dot{V}_{atem} \, dt = \int_{T_{insp}}^{T_{exsp}} \dot{V}_{atem} \, dt \tag{3}$$

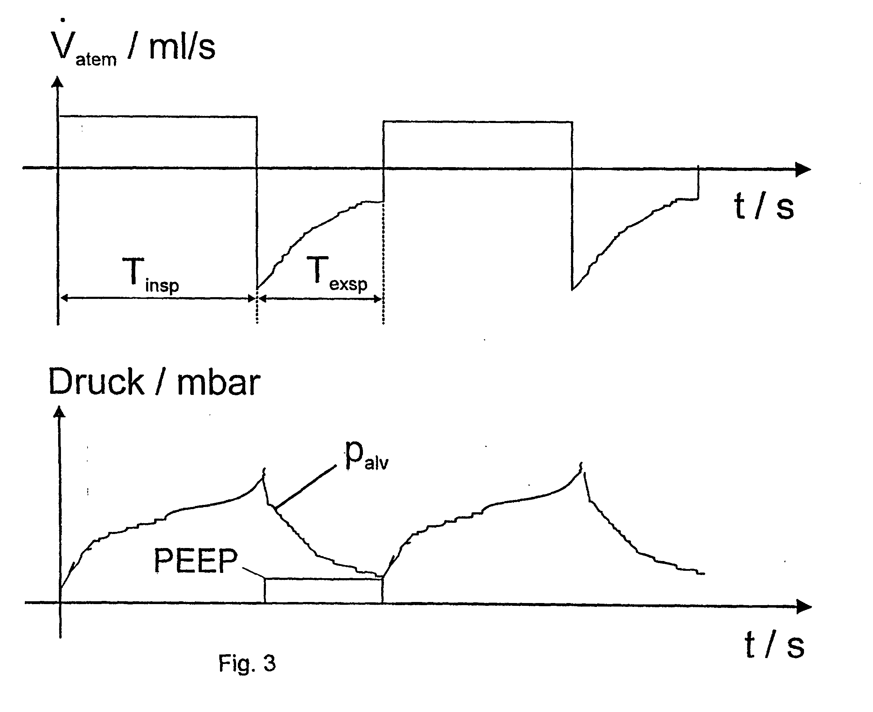

Bei der volumenkontrollierten Beatmung wird dem Patienten während der Inspiration ein konstanter Atemstrom aufgeprägt. Wie auch bei der druckkontrollierten Beatmung erfolgt die Exspiration passiv auf einen vorgewählten PEEP-Wert. Fig. 3 zeigt zur Veranschaulichung den Verlauf des Atemvolumenstroms sowie den Verlauf des alveolen Drucks bei der volumenkontrollierten Beatmung.

Im Gegensatz zu einer druckkontrollierten Beatmung kann bei einer volumenkontrollierten Beatmung ein bestimmtes (einstellbares) Tidalvolumen garantiert werden, weshalb diese Beatmungsform von machen Intensivmedizinern bevorzugt wird. Natürlich gilt im stationären Fall wiederum Gl. (3). Der Nachteil dieses Beatmungsmodus ist jedoch, dass die Amplitude von $p_{alv}$ nicht kontrolliert wird. Bei Patienten mit einer steifen Lunge können so unter Umständen Druckspitzen entstehen, die das Lungengewebe durch Überdehnung zum Reißen bringen können (sogenanntes "Barotrauma"). Ferner kann es bei Lungen mit inhomogenen Gewebeeigenschaften zu unerwünschten lokalen Luftverschiebungen innerhalb der Lunge kommen (sogenannte "Pendelluft").

Aus Leonhardt, S., Böhm, S. und Lachmann, B. "Optimierung der Beatmung beim akuten Lungenversagen durch Identifikation physiologischer Kenngrößen", Automatisierungstechnik (at), Vol. 46, No. 11, pp 532 - 539, 1998 sowie aus den Patentschriften US 5,660,170, US 5,738,090 und US 5,752,509 ist es grundsätzlich bekannt, die Öffnungs- und Schließdrücke bei einer kranken Lunge über den Sauerstoffpartialdruck paO2 zu bestimmen. Nach Identifizierung dieser Kennwerte werden die Beatmungsdrücke eines entsprechenden Beatmungsgerätes oberhalb des ermittelten Schließdrucks, bei dem die Lunge kollabiert, eingestellt. Diese Identifikation und Einstellung der Beatmungsdrücke erfolgt bereits automatisch. Ein Nachteil dieses bekannten Verfahrens besteht darin, dass die regelmäßige Messung des Sauerstoffpartialdrucks im Blut aufwendige Messgeräte erfordert und mitunter eine große Drift zur Folge hat. Zudem sind die zugehörigen Sensoren als Kathedersysteme sehr empfindlich und benötigen einen invasiven arteriellen Zugang, der die prinzipiell wünschenswerte Unversehrtheit des Körpers verletzt und eine potentielle Infektionsgefahr für den Patienten darstellt.

Aufgabe der Erfindung ist es daher, Einstellungsparameter für die künstliche Beatmung auf einfache und für den Patienten schonende Weise bereitzustellen.

Diese Aufgabe wird durch Vorrichtungen nach den Ansprüchen 1, 3 und 4 gelöst. Unter Verwendung der erfindungsgemäß ermittelten Einstellungsparameter wird gemäß Anspruch 5 außerdem eine Vorrichtung zur automatischen künstlichen Beatmung der menschlichen Lunge bereitgestellt.

Die Erfindung beruht auf der Erkenntnis, dass die Sättigung des Blutes $SO_2$ und/oder die Endtidale $CO_2$-Konzentration im Atemstrom und/oder das $CO_2$-Minutenvolumen im Atemstrom einfach extrakorporal gemessen werden können und kombiniert oder einzeln als Parameter zur Bestimmung des alveolären Öffnens und/oder des alveolären Schließens der Lunge herangezogen werden können. Ein invasiv arterieller Zugang ist für alle drei Parameter nicht erforderlich. Vielmehr können die Parameter extrakorporal gemessen werden und gleichzeitig auch als Feedback-Signale zur automatischen künstlichen Beatmung der Lunge verwendet werden.

Neben der bekannten Verwendung des Sauerstoffpartialdruckes im Blut kann auch die Blutsättigung selber als Messwert verwendet werden, um das alveoläre Öffnen oder das alveoläre Schließen der Lunge zu bestimmen. Beispielsweise kann der Blutsättigungswert seitens des Beatmungsgerätes zunächst auf 80% eingestellt werden, wobei bei allmählicher Erhöhung des Beatmungsdrucks das alveoläre Öffnen der Lunge durch einen signifikanten Anstieg in der Blutsättigung erkannt werden kann. Dementsprechend kann bei nachfolgendem Absinken des Beatmungsdruk-

kes das alveoläre Schließen durch ein plötzliches Absinken der Blutsättigung erkannt werden.

Die direkte Auswertung der Blutsättigung hat allerdings den Nachteil, dass der Sättigungswert beim Absinken des Beatmungsdruckes unter einen zulässigen Grenzwert fallen kann, wodurch für den Patienten lebensbedrohliche Situationen entstehen können. Nach einer bevorzugten Ausführungsform ist deshalb vorgesehen, dass die Blutsättigung in einem unterlagerten Regelkreis durch Regelung der von dem Beatmungsgerät abgegebenen Sauerstoffkonzentration näherungsweise auf einem vorgegebenen Sättigungswert gehalten wird. Wird sodann der Beatmungsdruck verändert, liefert die resultierende Regelgröße, also der resultierende Verlauf der eingestellten Sauerstoffkonzentration am Beatmungsgerät, ein signifikantes Signal zur Erkennung des alveolären Öffnens oder des alveolären Schließens der Lunge. Wird demnach der Beatmungsdruck von einem niedrigen Druck beginnend kontinuierlich erhöht, so sinkt beim alveolären Öffnen der Lunge die eingestellte Sauerstoffkonzentration am Beatmungsgerät signifikant ab, während beim darauffolgenden Verringern des Beatmungsdruckes die eingestellte Sauerstoffkonzentration am Beatmungsgerät sich wieder signifikant erhöht. Zur automatischen Signalauswertung der eingestellten Sauerstoffkonzentration kann vorgesehen sein, dass beim kontinuierlichen Erhöhen des Beatmungsdruckes ein alveoläres Öffnen der Lunge festgestellt wird, wenn die Steigung des resultierenden Verlaufs der eingestellten Sauerstoffkonzentration ein negatives Minimum erreicht. Umgekehrt kann bei kontinuierlicher Verringerung des Beatmungsdruckes ein alveoläres Schließen der Lunge festgestellt werden, wenn die Steigung des resultierenden Verlaufs der eingestellten Sauerstoffkonzentration ein positives Maximum erreicht.

Nach einer weiteren erfindungsgemäßen Lösung wird die endtidale $CO_2$-Konzentration im Atemstrom gemessen, um das alveoläre Öffnen oder das alveoläre Schließen der Lunge zu erkennen. Zusätzlich oder alternativ kann auch das $CO_2$-Minutenvolumen im Atemstrom gemessen werden, um ein alveoläres Öffnen oder ein alveoläres Schließen der Lunge zu erkennen. Das $CO_2$-Minutenvolumen ([ml $CO_2$/min]) kann zum Beispiel mit Hilfe einer kontinuierlichen Messung der $CO_2$-Konzentration im Atemstrom und aus dem Atemflusssignal und der Atemfrequenz berechnet werden. Bei Änderung des Beatmungsdruckes zeigen die $CO_2$-Konzentration und das $CO_2$-Minutenvolumen einen ähnlichen Verlauf. Dementsprechend kann bei Erhöhung des Beatmungsdruckes beginnend von einem niedrigen Wert beim alveolären Öffnen der Lunge ein signifikanter Anstieg der $CO_2$-Konzentration und des $CO_2$-Minutenvolumens festgestellt werden. Wird sodann der Beatmungsdruck kontinuierlich verringert, kann das alveoläre Schließen der Lunge anhand eines plötzlichen Abfalls der $CO_2$-Konzentration bzw. des $CO_2$-Minutenvolumens erkannt werden. Eine automatische Signalverarbeitung bei Erhöhung des Beatmungsdruckes zur Feststellung des alveolären Öffnens der Lunge kann beispielsweise auf dem Kriterium basieren, dass die positive Steigung des resultierenden Verlaufs der gemessenen endtidalen $CO_2$-Konzentration und/oder des gemessenen $CO_2$-Minutenvolumens im Atemstrom eine maximale Änderung erreicht. Sobald also die zweite Ableitung bei positiver erster Ableitung der jeweiligen Kurve in Abhängigkeit vom Beatmungsdruck ein Maximum annimmt, kann auf ein alveoläres Öffnen der Lunge geschlossen werden. Umgekehrt kann bei kontinuierlicher Verringerung des Beatmungsdruckes auf ein alveoläres Schließen der Lunge geschlossen werden, wenn die negative Steigung des resultierenden Verlaufs der gemessenen endtidalen $CO_2$-Konzentration und/oder des gemessenen $CO_2$-Minutenvolumens im Atemstrom eine maximale Änderung erreicht, d. h. die zweite Ableitung bei negativer erster Ableitung ein Maximum annimmt.

Die Verfahren zur Bestimmung des alveolären Öffnens oder des alveolären Schließens der Lunge können dazu herangezogen werden, um eine erfindungsgemäße Vorrichtung zur automatischen künstlichen Beatmung der menschlichen Lunge bereitzustellen, wobei dann über eine Steuereinheit die Beatmungsparameter des Beatmungsgerätes einstellbar sind, und wobei der dem alveolären Öffnen entsprechende Beatmungsdruck und/oder der dem alveolären Schließen entsprechende Beatmungsdruck von einer Recheneinheit in mindestens eine Regelgröße umgesetzt und diese Regelgröße der Steuereinheit derart zugeführt wird, dass ein maximaler Gasaustausch bei minimaler mechanischer Belastung der Lunge stattfindet.

Weitere Einzelheiten und Vorteile der Erfindung werden anhand der in den Zeichnungen dargestellten Ausführungsbeispiele näher erläutert.

Fig. 1    zeigt den Bronchialbaum des Menschen und eine Ausschnittsvergrößerung von Alveolen,

Fig. 2    zeigt den Verlauf des Beatmungsdrucks und des alveolen Drucks sowie den Verlauf des Atemvolumenstroms bei der druckkontrollierten Beatmung,

Fig. 3    zeigt den Verlauf des Atemvolumenstroms sowie den Verlauf des alveolen Drucks bei der volumenkontrollierten Beatmung,

Fig. 4    zeigt ein Blockschaltbild mit Signalflüssen zur Modellbildung der Beatmung,

Fig. 5    zeigt ein elektrisches Ersatzschaltbild der Lungenmechanik beim spontanatmenden Menschen,

Fig. 6      zeigt ein elektrisches Ersatzschaltbild der Lungenmechanik bei der druckkontrollierten Beatmung,

Fig. 7      zeigt pV-Diagramme bei gesunder (links) und schwerkranker (rechts) Lunge,

Fig. 8      zeigt den arteriellen Sauerstoffpartialdruck bei gesunder (links) und schwerkranker (rechts) Lunge,

Fig. 9      zeigt Druckrampen zur Identifikation des Großsignalverhaltens einer schwerkranken Lunge auf der Basis des Sauerstoffpartialdrucks,

Fig. 10     zeigt die Anwendung des Verfahrens gemäß Fig. 9 für eine schonende Dauerbeatmung,

Fig. 11     zeigt ein erstes Ausführungsbeispiel einer erfindungsgemäßen Vorrichtung zur automatischen Dauerbeatmung auf der Basis der endtidalen $CO_2$-Konzentration und/oder des $CO_2$-Minutenvolumens,

Fig. 12     zeigt ein schematisches Schaubild zur Erläuterung der Bestimmung des $CO_2$-Minutenvolumens,

Fig. 13     zeigt Druckrampen zur Identifikation des Großsignalverhaltens einer schwerkranken Lunge auf der Basis der endtidalen $CO_2$-Konzentration und/oder des $CO_2$-Minutenvolumens,

Fig. 14     zeigt den Zusammenhand zwischen Sauerstoffpartialdruck $paO_2$, physikalisch gelöstem $O_2$ und der Sättigung,

Fig. 15     zeigt ein zweites Ausführungsbeispiel einer erfindungsgemäßen Vorrichtung zur automatischen Dauerbeatmung auf der Basis der Blutsauerstoffsättigung,

Fig. 16     zeigt einen unterlagerten Regelkreis zur automatischen Einstellung der Sauerstoffkonzentration am Beatmungsgerät bei vorgegebener Blutsauerstoffsättigung und

Fig. 17     zeigt Druckrampen zur Identifikation des Großsignalverhaltens einer schwerkranken Lunge auf der Basis der momentan eingestellten Sauerstoffkonzentration am Beatmungsgerät gemäß Fig. 16.

Fig. 1 bis Fig. 3 wurden bereits im Zusammenhang mit der Erläuterung des Standes der Technik beschrieben.

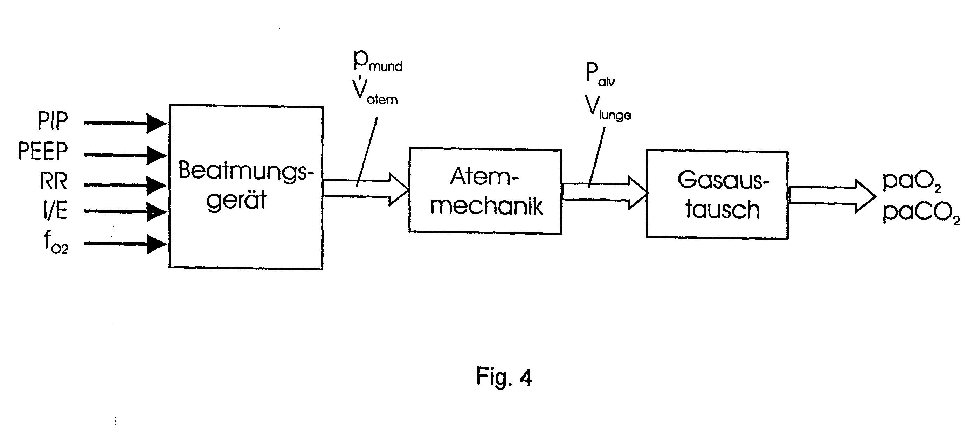

Fig. 4 zeigt ein Blockschaltbild mit Signalflüssen zur Modellbildung der Beatmung. Die Modellbildung der Beatmung hat zum Ziel, anhand der erfindungsgemäßen Verfahren möglichst genau die Lunge identifizieren zu können, um anhand dessen wiederum eine automatische Beatmung mit maximalem Gasaustausch bei minimaler mechanischer Belastung der Lunge durchführen zu können.

Zum besseren Verständnis eines Prozesses und zur Auslegung von Regelungen oder Steuerungen empfiehlt es sich generell, ein für die Applikation geeignetes Prozessmodell zu entwicklen. Eine systemtheoretische Modellbildung der beatmeten Lunge sollte die folgenden drei Teilsysteme beinhalten: "Beatmungsgerät", "Atemmechanik" und "Gasaustausch".

Fig. 4 zeigt ein Blockschaltbild mit Signalflüssen zur Modellbildung der Beatmung. Die Eingangsgrößen dieses Prozesses sind neben den Beatmungsdrücken PIP und PEEP die Atemfrequenz RR, das Einatmungs- zu Ausatmungsverhältnis I/E und die Sauerstoffkonzentration $f_{O2}$ (20 ... 100%). Die Ausgangsgrößen sind die im arteriellen Blut herrschenden Partialdrücke $paO_2$ und $paCO_2$, während das aktuelle Füllungsvolumen $V_{lunge}$ und der alveoläre Druck $p_{alv}$ innere Zustände des Prozesses sind, über die bisher allerdings wegen fehlender messtechnischer Voraussetzungen kaum Aussagen gemacht werden konnten.

Insbesondere die Teilsysteme "Atemmechanik" und "Gasaustausch" sind stark nichtlinear. Es empfiehlt sich daher, sowohl das Kleinsignal- wie auch das Großsignalverhalten zu analysieren.

Bei der Analyse des Kleinsignalverhaltens ist zu berücksichtigen, dass die Lunge über ein sich zunehmend verzweigtes Röhrensystem (den sogenannten "Bronchialbaum") belüftet wird. In diesem aus bis zu 23 Generationen bestehenden Röhrensystem ist ein großer Teil des Strömungswiderstandes $R_{ström}$ (resistance) lokalisiert, insbesondere in den Generationen 3-6.

Der zweite atemmechanische Parameter der Lunge, die Volumendehnbarkeit $C_{rs}$, wird hingegen durch die elastischen Eigenschaften der peripheren Gewebestrukturen bestimmt. Sie wird auch als "compliance" bezeichnet.

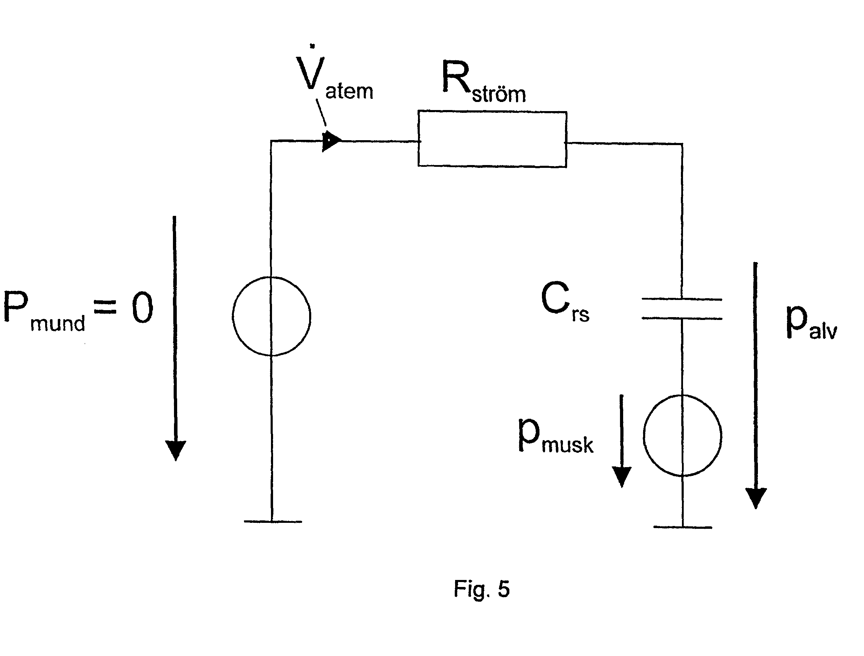

Fig. 5 zeigt ein elektrisches Ersatzschaltbild der Lungenmechanik beim spontanatmenden Menschen. Da der Munddruck eines spontanatmenden Menschen gleich dem Atmosphärendruck ist, gilt $p_{mund} = 0$. Aus dem Spannungsumlauf ergibt sich für die Lungenmechanik die folgende Differentialgleichung:

$$p_{musk}(t) + \frac{1}{C_{rs}} \cdot (V_{lunge}(t) - V_{lunge,0})$$

$$+ R_{ström} \cdot \dot{V}_{atem}(t) = p_{mund} = 0 \tag{4}.$$

Hierbei ist $p_{musk}$ der durch die Atemmuskulatur während der Inspiration hervorgerufene (Unter-)Druck und $V_{lunge,0}$ das in der Lunge befindliche Ruhe-Volumen. Die Dynamik dieses Systems wird durch die respiratorische Zeitkonstante

$$T_{rs} = R_{ström} \cdot C_{rs} \tag{5}$$

beschrieben. Für die Resistance und die Compliance gelten allgemein die folgenden Definitionsgleichungen:

$$R_{ström} = \frac{p_{mund} - p_{alv}}{\dot{V}_{atem}} \tag{6}$$

und

$$C_{rs} = \frac{V_{lunge} - V_{lunge,0}}{p_{alv} - p_{musk}} \tag{7}$$

Beim gesunden Erwachsenen liegen typische Werte bei $R_{ström}$ = 2 ... 4 mbar s/l und $C_{rs}$ = 230 ... 290 ml/mbar. Beide Parameter ändern sich jedoch stark mit dem Füllungszustand der Lunge und sind somit eine Funktion des Arbeitspunktes.

Beim beatmeten Patienten liegen andere Druckverhältnisse vor. Aufgrund einer medikamentösen Muskel-Relaxation ist in der Regel $p_{musk}$ = 0, dafür wird nun ein von 0 verschiedener Munddruck appliziert. Durch den in der Atemröhre liegenden Schlauch ("Tubus") entsteht ein zusätzlicher Strömungswiderstand, der in der Regel turbulent ist und durch einen quadratischen Term berücksichtigt werden kann. Daher ändert sich Gl. (4) zu

$$p_{mund}(1) = \frac{1}{C_{rs}} \cdot (V_{lunge}(t) - V_{lunge,0}) + R_{ström} \cdot \dot{V}_{atem}(t)$$

$$+ R_{tubus} \cdot \dot{V}_{atem}(t) \cdot |\dot{V}|_{atem}(t)| \tag{8}.$$

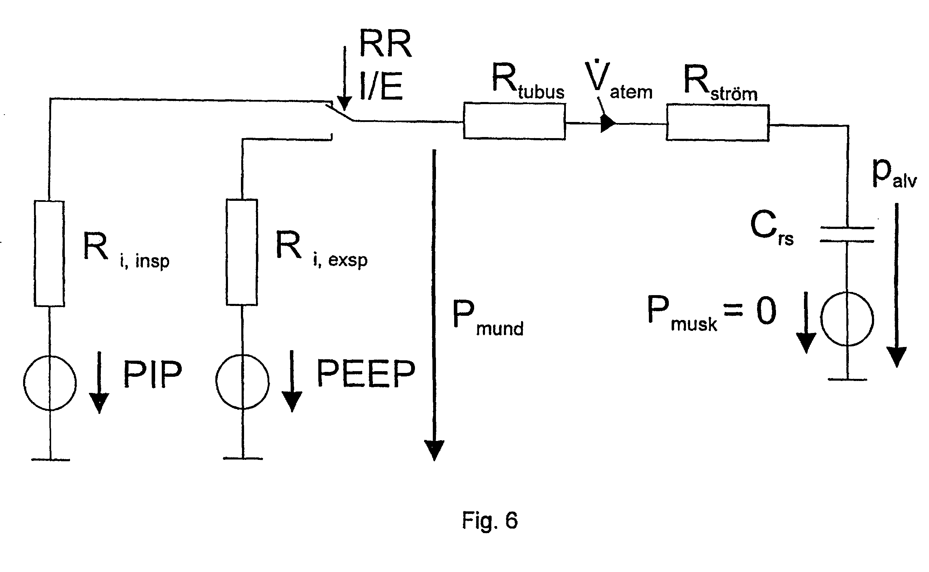

Fig. 6 zeigt ein elektrisches Ersatzschaltbild der Lungenmechanik bei der druckkontrollierten Beatmung. Im Alveolus setzen sich die Partialdrücke der einzelnen Gasfraktionen nach dem Gesetz von Dalton zum Gesamtdruck $p_{alv}$ gemäß

$$p_{alv} = p_{alv,O_2} + p_{alv,CO_2} + p_{alv,N_2} + p_{alv,H_2O} \tag{9}$$

zusammen. Hierbei entsteht der Wasserdampfpartialdruck $p_{alv,H_2O}$ durch Anfeuchtung in den Atemwegen. Man erkennt, dass der alveoläre Sauerstoff-Partialdruck selbst bei Beatmung mit 100%igem Sauerstoff (d.h., $p_{alv,N_2}$ = 0 mmHg) in Ruhe um den Wasserdampfpartialdruck und den aktuellen Kohlendioxidpartialdruck $p_{alv,CO_2}$ geringer ist als der Atmosphärendruck. Wegen $p_{alv,H_2O}$ = 47 mmHg und $p_{alv,CO_2}$ = 39 mmHg (jeweils bei 37 C) gilt somit

$$p_{alv,O_2} \leq 674 \text{ mmHg} \tag{10}.$$

Bei Normaldruckbeatmung kann dementsprechend auch der arterielle $paO_2$ niemals über diesen Wert ansteigen.

Der Gasaustausch erfolgt gemäß dem 1. Fick'schen Diffusionsgesetz. So gilt z.B. für den Sauerstoffvolumenstrom aus den Alveolen ins Blut

$$\frac{dV_{O_2}}{dt} = \frac{k \cdot A_{diff}}{l_{diff}} \cdot (p_{alv.O_2} - paO_2)$$

$$= R_{diff} \cdot (p_{alv,O_2} - paO_2) \,. \tag{11}$$

Der diffundierende Volumenstrom ist daher neben dem Partialdruckgefälle direkt abhängig von der effektiven Gasaustauschfläche $A_{diff}$ und umgekehrt proportional zur effektiven Diffustionsstrecke $l_{diff}$. Beim gesunden Menschen gleichen sich die Konzentrationsunterschiede zwischen den alveolären Partialdrücken und den im arteriellen Blut herrschenden Partialdrücken ($paO_2$ und $paCO_2$) in kurzer Zeit (« 1 s) aus, da der Diffusionskoeffizient K für $CO_2$ etwa 20 mal größer als für $O_2$ ist.

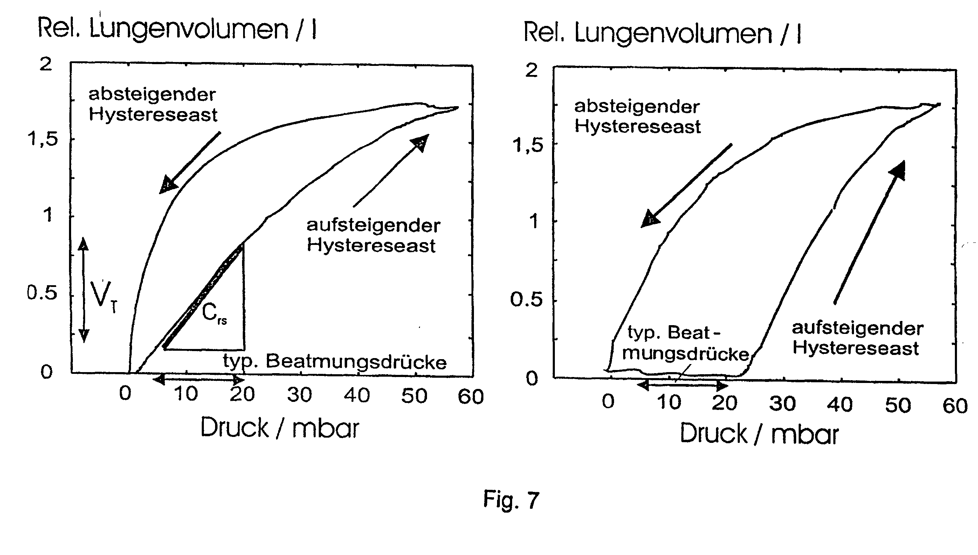

Das mechanische Großsignalverhalten einer Lunge lässt sich am besten durch ein pV-Diagramm beschreiben. Fig. 7 zeigt hierzu pV-Diagramme bei gesunder (links) und schwerkranker (rechts) Lunge, wobei hier nicht das absolute Lungenfüllungsvolumen, sondern das auf das beim Druck Null herrschende Volumen bezogene relative Volumen dargestellt ist. In diesem Diagramm lässt sich die Compliance $C_{rs}$ auch durch

$$C_{rs} = \frac{V_T}{PIP - PEEP} \tag{12}$$

annähern (sogenannte "statische" Compliance). Man erkennt, dass eine gesunde Lunge (links) über dem gesamten Druckbereich eine von Null verschiedene Compliance hat und bei typischen Beatmungsdrücken (z.B. PIP = 20 mbar, PEEP = 5 mbar) mit einem ausreichenden Tidalvolumen $V_T$ versorgt werden kann.

Bei der schwerkranken Lunge (rechts) hingegen ist die Hysterese anders geformt und stärker ausgeprägt. Insbesondere auf dem aufsteigenden Schenkel der Hysterese erreicht man bei typischen Beatmungsdrücken nur ein geringes Tidalvolumen. In diesem Bereich ist ein Großteil der Alveolen kollabiert und steht für den Gasaustausch nicht zur Verfügung.

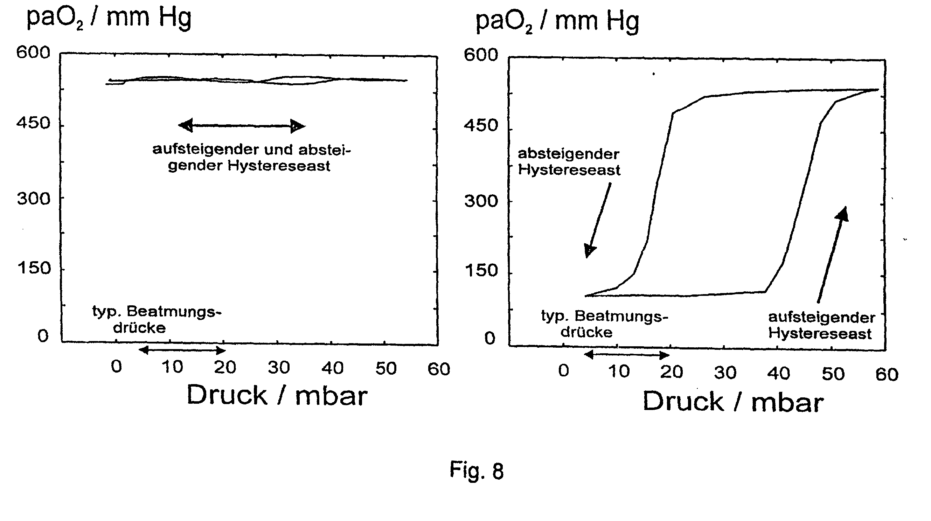

Noch deutlicher wird dies, wenn man das Großsignalverhalten des arteriellen Sauerstoff-Partialdrucks betrachtet. Fig. 8 zeigt den arteriellen Sauerstoffpartialdruck bei gesunder (links) und schwerkranker (rechts) Lunge. Während es bei der gesunden Lunge (links) für $paO_2$ gar keine Hysterese gibt und die Wahl der Beatmungsdrücke unkritisch zu sein scheint, findet sich bei einer schwerkranken Lunge (rechts) eine noch ausgeprägtere Hysterese als im zugehörigen pV-Diagramm. Die Gasaustauschfläche $A_{diff}$ ist hier in manchen Bereichen so stark reduziert, dass bei typischen Beatmungsdrücken nur bei hochprozentiger Sauerstoffgabe (90 ... 100%) ein mit dem Leben vereinbarer $paO_2$ (> 80 mmHg) erreicht werden kann.

Eine solche Lunge lässt sich nur dann sinnvoll beatmen, wenn sie zunächst mit großen Drücken "geöffnet" wird, um anschließend auf dem absteigenden Schenkel der Hysterese auch bei niedrigeren Drücken ein ausreichendes Ventilationsvolumen zu erzielen.

Die auch bei der gesunden Lunge vorhandene, aber deutlich geringere Hysterese im pV-Diagramm (Fig. 7, links) spiegelt die Tatsache wieder, dass es auch physiologischerweise einen gewissen Anteil an kollabierten Alveolen gibt, den man zwar druckgesteuert rekrutieren kann, der aber für das Erreichen des maximalen Sauerstoff-Partialdrucks nicht notwendig ist.

Für eine dem jeweiligen Patienten angepasste Beatmung müssen das dynamische und das statische Verhalten der Lunge bekannt sein. Da sie sich unter Umständen innerhalb eines Tages deutlich verändern können, ist es wichtig, die Lunge jedes Patienten so oft wie nötig zu identifizieren.

Hinsichtlich des Kleinsignalverhaltens kann bei nicht zu großen Flüssen der durch den Tubus verursachte Widerstandsanteil als laminar betrachtet und $R_{ström}$ zugeschlagen werden. Der Strömungswiderstand und die Compliance lassen sich z.B. aus dem exspiratorischen Teil der Atemflusskurve bestimmen. Betrachtet man $R_{ström}$ und $C_{rs}$ während der Exspiration als konstant, so gelten folgende Schätzgleichungen

$$\hat{R}_{ström} = \frac{PIP - PEEP}{\dot{V}_{atem,max}} \tag{13}$$

und

$$\hat{C}_{rs} = \frac{\hat{T}_{rs}}{\hat{T}_{str\ddot{o}m}} \qquad (14).$$

Hierbei wird die respiratorische Zeitkonstante $\hat{T}_{rs}$ aus dem gemessenen exspiratorischen Flussverlauf nach der Least Squares (LS) Methode bestimmt. Wie sich gezeigt hat, ist der diagnostische Wert dieser beiden Parameter alleine nicht sehr hoch. Zwar ist es richtig, dass sie im Krankheitsfall spezifisch verändert sein können (z.B. ist $R_{str\ddot{o}m}$ bei einer Bronchitis in der Regel erhöht), leider ändern sie sich aber auch mit dem Füllungszustand der Lunge, d.h. $R_{str\ddot{o}m}$, $C_{rs} = f (V_{lunge})$. Hinzu kommt, dass nach Fig. 7 zumindest für $C_{rs}$ auch eine druckabhängige Hysterese nachweisbar ist. Damit verändert sich $C_{rs}$ auch noch als Funktion der zeitlichen Abfolge der Beatmungsdrücke. Durch diese Tatsache ist die isolierte Interpretation des Kleinsignalverhaltens am Arbeitspunkt nicht möglich. Für eine optimale Beatmung ist es daher wesentlich, auch das Großsignalverhalten der Lunge zu kennen.

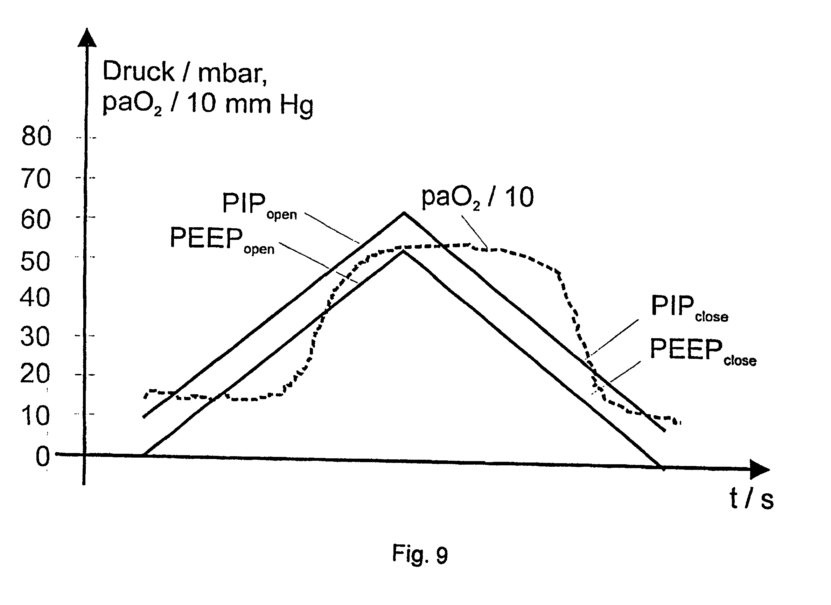

Zur Identifikation des Großsignalverhaltens eignet sich z.B. eine Druckrampe, mit der die Hysterese der Lunge einmal vollständig durchfahren werden kann. Fig. 9 zeigt hierzu als Beispiel Druckrampen zur Identifikation des Großsignalverhaltens einer schwerkranken Lunge auf der Basis des Sauerstoffpartialdruckes. Die Verwendung des Sauerstoffpartialdruckes zur Bestimmung des alveolären Öffnens und des alveolären Schließens der Lunge ist grundsätzlich aus der bereits eingangs erwähnten VEröffentlichung Leonhardt, S., Böhm, S. und Lachmann B., "Optimierung der Beatmung beim akuten Lungenversagen durch Identifikation physiologischer Kenngrößen", Automatisierungstechnik (at), Vol. 46, No. 11, pp 532 - 539, 1998 bekannt. Mit den erfindungsgemäßen Verfahren kann sinngemäß die gleiche Identifikation durchgeführt werden, was weiter unten noch erläutert werden wird.

Zur Bestimmung des Öffnungs- und Schließdrucks dient die bekannte Definition, dass eine kranke Lunge als "sicher offen" anzusehen ist, wenn $paO_2 > 450$ mmHg ist (bei Beatmung mit 100% Sauerstoff). In gleicher Weise gilt eine Lunge als "sicher geschlossen" (zu einem großen Teil kollabiert), wenn $paO_2 < 300$ mmHg ist. Das Hystereseverhalten der Lunge bezüglich $paO_2$ ist durch die Öffnungs- und Schließdrücke vollständig bestimmt.

Zur Dauerbeatmung kann die Lunge sodann in einem nächsten Schritt wieder geöffnet werden, und zwar anhand des Öffnungsdruckes, der vorher bestimmt wurde, um danach eine Dauerbeatmung knapp oberhalb dem Schließdruck fortzusetzen.

Gemäß Fig. 9 wird dabei zweckmäßigerweise nicht nur mit einem Beatmungsdruck, sondern mit zwei Beatmungsdrücken gearbeitet, die typischerweise an einem Beatmungsgerät einzustellen sind, nämlich zum einen der eingangs erläuterte Einatmungsdruck (PIP) und zum anderen der eingangs erläuterte Ausatmungsgegendruck (PEEP). Anhand dieser Beatmungsdrücke kann also nach der Identifikation der Lunge eine automatische Dauerbeatmung erfolgen.

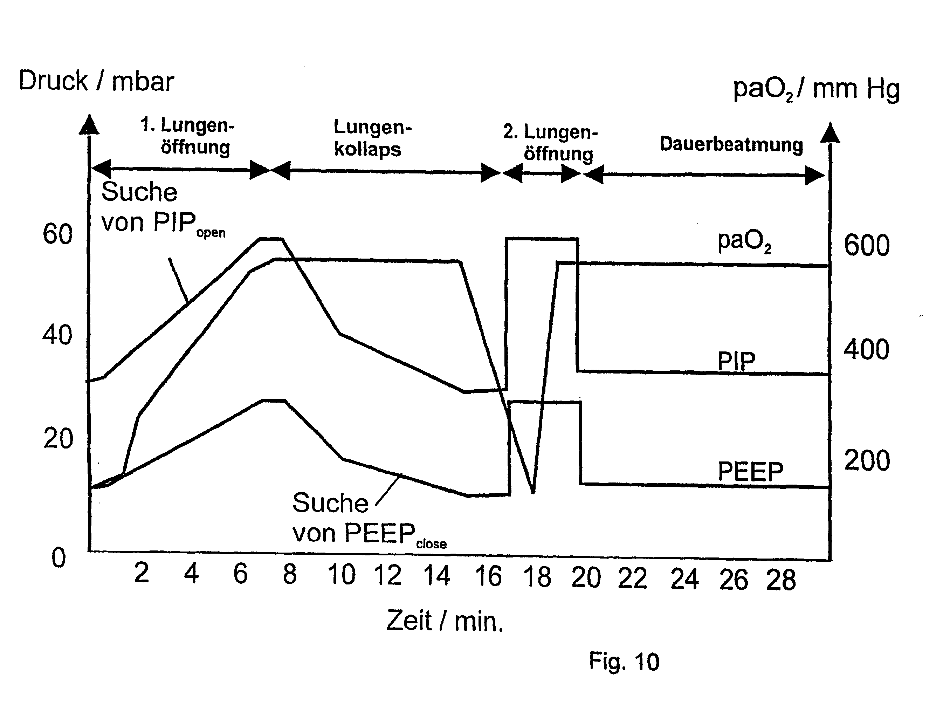

Fig. 10 zeigt demgemäß die Anwendung des Verfahrens gemäß Fig. 9 für eine schonende Dauerbeatmung. Nach Bestimmung des Öffnungs- und Schließdruckes sowohl für den Einatmungsdruck PIP, als auch für den Ausatmungsgegendruck PEEP wird die Lunge erneut geöffnet und anschließend bei minimal möglichem Druck etwas oberhalb der Schließdrücke ventiliert. Zum Beispiel kann man den Ausatmungsgegendruck wie folgt wählen:

$$PEEP = PEEP_{close} + 2 \text{ mmHg} \qquad (15)$$

und weiterhin den Einatmungsdruck PIP dergestalt, dass das Atemzugvolumen in einem günstigen Bereich liegt. Aber auch andere Werte sind möglich, sobald sie oberhalb der Schließdrücke liegen.

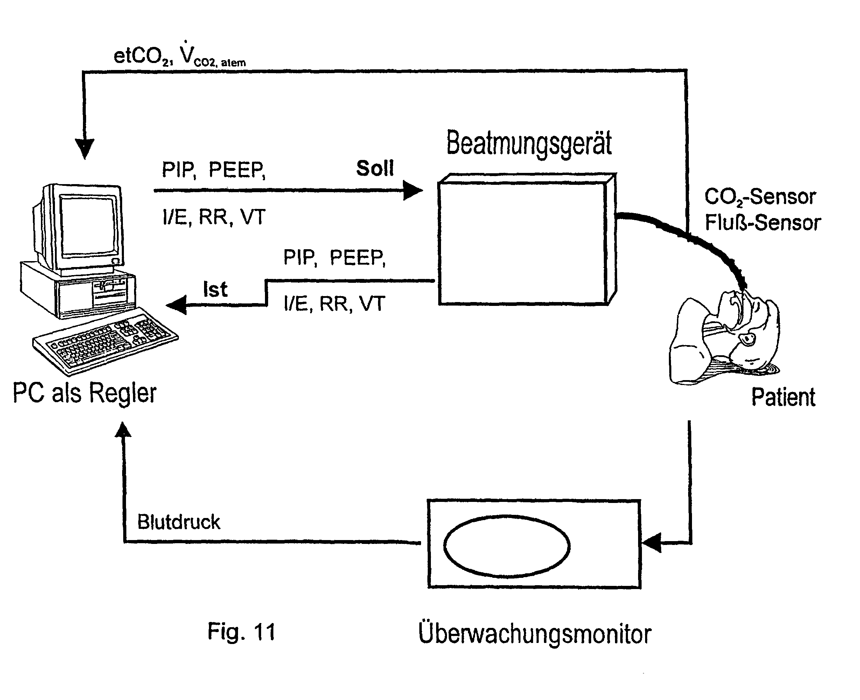

Anstelle des invasiv zu messenden $paO_2$ benutzt das erfindungsgemäße Verfahren nach einem ersten Ausführungsbeispiel die nicht-invasiv im Atemstrom messbare endtidale $CO_2$-Konzentration und das $CO_2$-Minutenvolumen als Feedback-Signale zur Ermittlung der optimalen Beatmungsparameter bei atelektatischen Lungen. Das Konzept kann im Sinne einer closed loop Regelung implementiert werden. Fig. 11 zeigt hierzu ein erstes Ausführungsbeispiel einer erfindungsgemäßen Vorrichtung zur automatischen Dauerbeatmung auf der Basis der endtidalen $CO_2$-Konzentration und/oder des $CO_2$-Minutenvolumens.

Hierbei kann die automatische Einstellung der Beatmungsparameter mit Hilfe externer Geräte (z.B. einem zusätzlichen Personal Computer) oder durch entsprechende Integration der Algorithmen in das Beatmungsgerät realisiert werden.

Das $CO_2$-Minutenvolumen (in [ml $CO_2$/min] kann z.B. mit Hilfe einer kontinuierlichen Messung der $CO_2$-Konzentration [$CO_2$] im Atemstrom und aus dem Atemflusssignal und der Atemfrequenz gemäß

$$\dot{V}_{CO_2Atem} = RR \cdot \int_{0}^{T_i} [CO_2](t) \cdot \dot{V}_{Atem}(t) DT \qquad (16)$$

berechnet werden. $T_e$ ist die Exspirationszeit. Bilanziert man die $CO_2$-Produktion und -Elimination im Körper, so ergibt sich

$$V_{CO_2,speicher}(t) = V_{CO_2,speicher}(0) + \int_{0}^{t} (\dot{V}_{CO_2,prod} - \dot{V}_{CO_2,Atem}) dt \qquad (17).$$

Bei einem relaxierten Patienten kann - zumindest für den hier betrachteten Zeitraum, der im Minuten- bis wenige Stundenbereich liegen soll - von einer mehr oder weniger konstanten $CO_2$-Produktion im Körper ausgegangen werden. Typische Werte für die $CO_2$-Produktion liegen bei 5 ... 7 ml/kg min. Längerfristig auf den $CO_2$-Haushalt einwirkende physiologische Regulationsmechanismen wie die pH-Wert-Regelung über die Niere können für die hier betrachteten Zeiträume vernachlässigt werden.

Ist die $CO_2$-Elimination infolge eines Teilkollapses der Lunge unzureichend, so wird die im Körperwasser gespeicherte $CO_2$-Menge und auch der arterielle $paCO_2 = (fV_{CO2speicher})$ ansteigen.

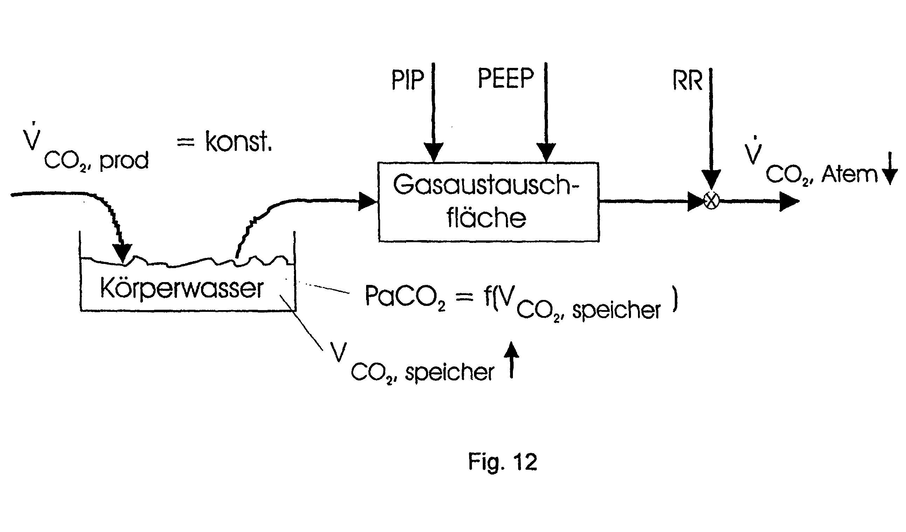

Fig. 12 zeigt ein schematisches Schaubild zur Erläuterung der Bestimmung des $CO_2$-Minutenvolumens.

Es ist bekannt, dass bei normalen Lungen mit geringen Atelektasen und kleiner mechanischer Hysterese durch die Vergrößerung von PIP - PEEP (und damit dem Tidalvolumen) oder RR mehr $CO_2$ ausgewaschen und damit der $paCO_2$ gesenkt werden kann (der $paCO_2$ kann aber auch über das I/E Verhältnis beeinflusst werden). Bei Lungen mit größeren Atelektasen lässt sich dies zur Identifikation der Hysterese-Kennwerte ausnutzen.

Zu Beginn der Identifikation empfiehlt es sich, zunächst einen "steady state" abzuwarten und RR temporär niedrig zu wählen, um den $paCO_2$ kurzfristig ansteigen zu lassen. Werden nun die Beatmungsdrücke langsam erhöht, so lässt sich die mechanische Öffnung der Lunge an einer plötzlich überschießenden endtidalen $CO_2$-Konzentration ($etCO_2$) und entsprechend erhöhtem $CO_2$-Minutenvolumen erkennen. Entsprechend sinkt jetzt $V_{CO2.speicher}$ ab. Im Unterschied zum $paO_2$ bleiben beide Signale während der offenen Phase jedoch nicht konstant, sonder sinken, da sich die $CO_2$-Körperspeicher über die vergrößerte Gasaustauschfläche entleeren.

Werden anschließend die Beatmungsdrücke reduziert, so erkennt man einen Lungenkollaps daran, dass das endtidale $CO_2$-Signal und das $CO_2$-Minutenvolumen wieder deutlich absinken.

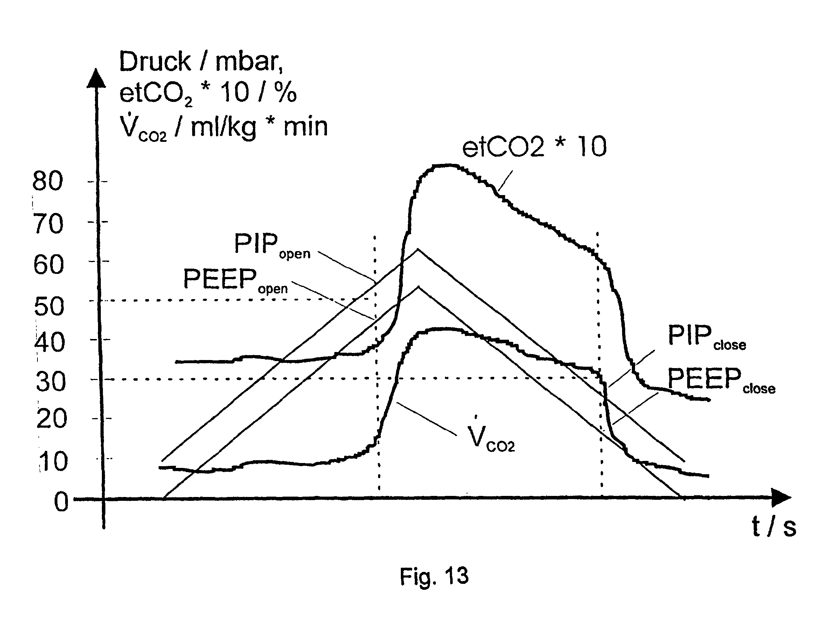

Fig. 13 zeigt Druckrampen zur Identifikation des Großsignalverhaltens einer schwerkranken Lunge auf der Basis der endtidalen $CO_2$-Konzentration und/oder des $CO_2$-Minutenvolumens.

Man beachte, dass die absolute Amplitude der Veränderungen des $etCO_2$-Signals und des Atemminutenvolumens auch eine Funktion des absoluten $paCO_2$ ist. Daher sollte vor Beginn dieser Identifikation und Therapie der Algorithmus durch eine einmalige Messung des $paCO_2$ kalibriert werden. Günstig ist ferner, wenn am Anfang der Therapie ein steady state vorliegt und der $paCO_2$ erhöht ist, z.B. durch temporäre Reduktion der Atemfrequenz RR.

Alternativ zu den absoluten Änderungen können auch nur die relativen Änderungen des $etCO_2$-Signals und des Atemminutenvolumens zur Identifikation der Öffnungs- und Schließdrücke herangezogen werden.

Nach der Identifikation der Öffnungs- und Schließdrücke wird die Lunge erneut durch Blähung geöffnet und anschließend oberhalb der Schließdrücke beatmet. Da bei offener Lunge der $etCO_2$ ein Mass für den $paCO_2$ ist, kann anschließend der $paCO_2$ auf Normalwerte ausgeregelt werden.

Nach einem zweiten erfindungsgemäßen Ausführungsbeispiel wird anstelle des invasiv zu messenden $paO_2$ die nicht-invasiv messbare Blutsauerstoffsättigung $SO_2$ als Feedback-Signal zur Ermittlung der optimalen Beatmungsparameter bei atelektatischen Lungen verwendet.

Zur Anwendung des Verfahrens wird vorausgesetzt, dass die Blutsauerstoffsättigung peripher messbar ist (d.h., der Patient ist nicht durch Schock o.ä. zentralisiert).

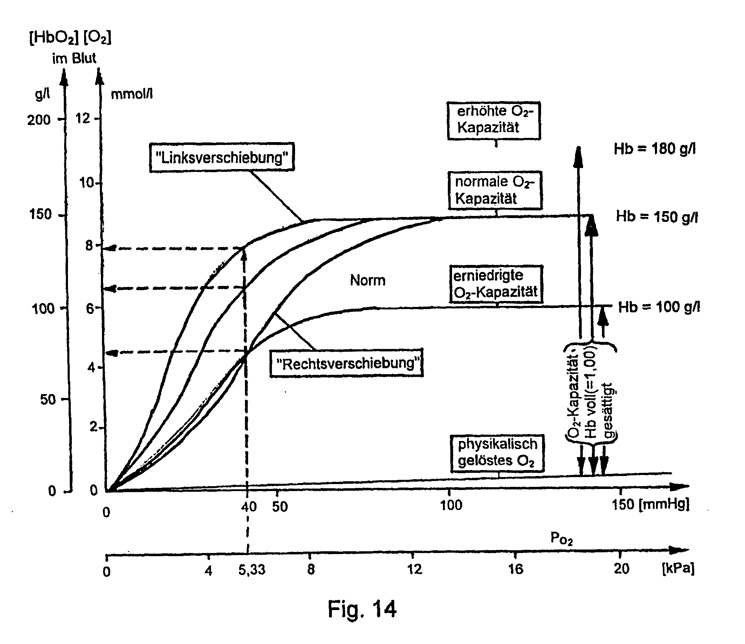

Fig. 14 zeigt den Zusammenhang zwischen Sauerstoffpartialdruck $paO_2$, physikalisch gelöstem $O_2$ und der Blutsauerstoffsättigung.

Dennoch wird der Sättigungssollwert sinnvollerweise so vorgegeben, dass $S_{O_2}$ im physiologischen Bereich (80 ... 100%) liegt, aber dennoch ausreichenden Signalhub besitzt. Ein typische Wert ist z.B. $S_{O_2,soll}$ = 90%.

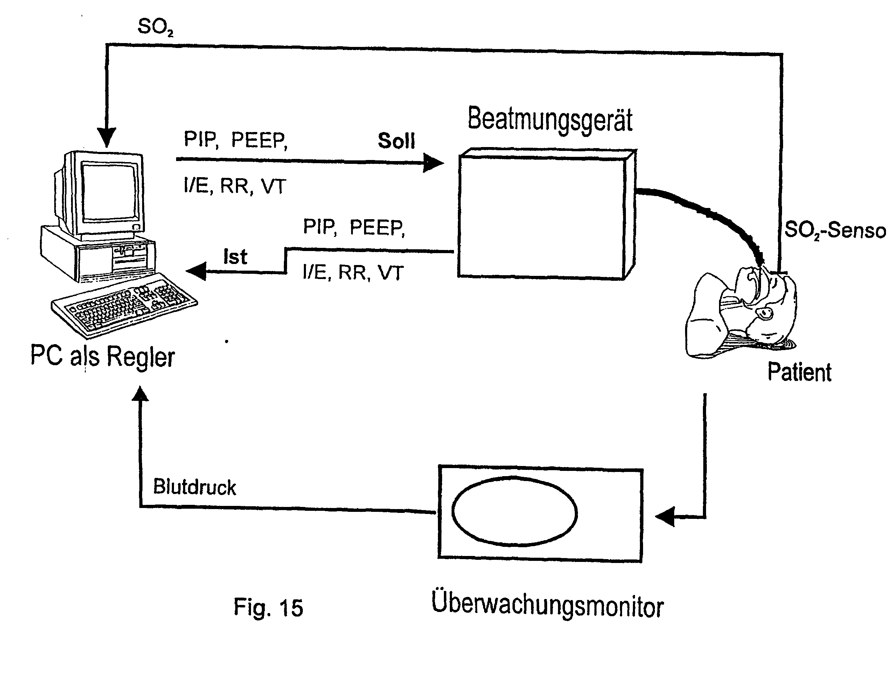

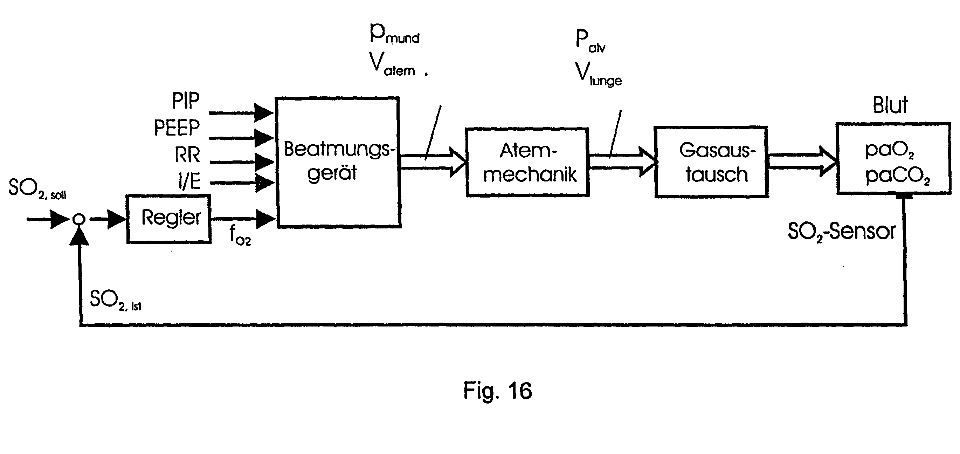

Fig. 15 zeigt ein zweites Ausführungsbeispiel der erfindungsgemäßen Vorrichtung zur automatischen Dauerbeatmung auf der Basis der Blutsauerstoffsättigung. Die automatische Einstellung der Beatmungsparameter kann mit Hilfe eines Blutsauerstoffsensors und weiterer externer Geräte oder durch entsprechende Integration der Algorithmen in das Beatmungsgerät realisiert werden. Wie bereits oben erläutert, wird in einer bevorzugten Ausführung nicht direkt der Wert der Blutsauerstoffsättigung verwendet, sondern statt dessen die durch einen unterlagerten Regelkreis eingestellte Sauerstoffkonzentration aufgrund eines vorgegebenen Blutsauerstoffsättigungswertes herangezogen. Fig. 16 zeigt einen derartigen unterlagerten Regelkreis zur automatischen Einstellung der Sauerstoffkonzentration am Beatmungsgerät bei vorgegebener Blutsauerstoffsättigung.

Vorzugsweise ist die Regelungsgüte der $S_{O_2}$-Regelung so groß, dass der $S_{O_2}$ auch bei Veränderung der anderen Einstellparameter des Beatmungsgerätes konstant gehalten werden kann.

Unter dieser Voraussetzung ist der $f_{O_2}$ ein Mass für den Anteil offener Alveolen in einer Lunge. Wird eine hohe Sauerstoffkonzentration benötigt (z.B. $f_{O_2}$ = 70% oder mehr), um $S_{O_2}$ = 90% zu erreichen, ist die Lunge zu einem großen Teil kollabiert. Wird hingegen nur eine niedrige Sauerstoffkonzentration benötigt, so ist die Lunge relativ offen.

Diese Tatsache lässt sich zur Identifikation der Hysterese-Parameter von atelektatischen Lungen, wie sie z. B. beim ARDS auftreten (siehe oben), benutzen. In einer bevorzugten Ausführung der Erfindung werden während der Regelung der Sauerstoffsättigung zur Identifikation der Lunge die Beatmungsdrücke PIP und PEEP verändert.

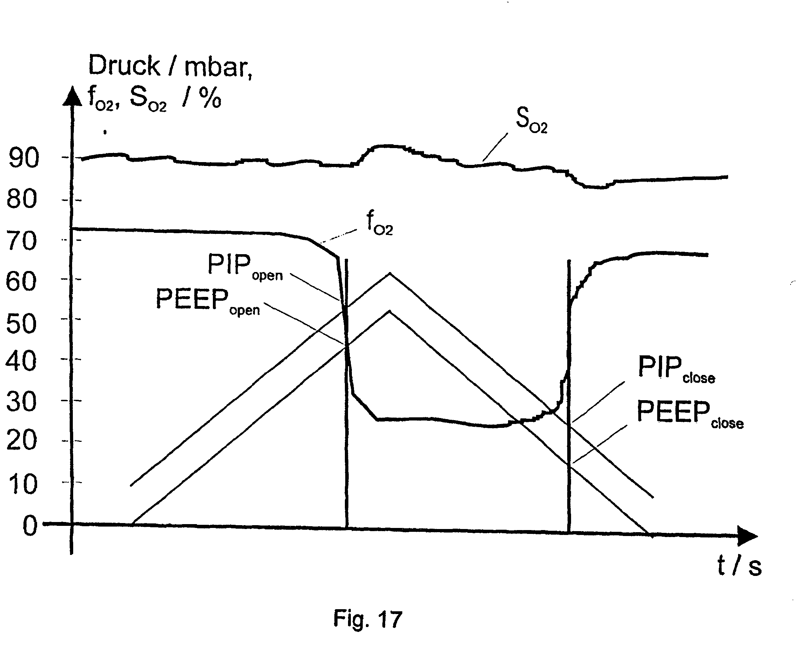

Fig. 17 zeigt Druckrampen zur Identifikation des Großsignalverhaltens einer schwerkranken Lunge auf der Basis der momentan eingestellten Sauerstoffkonzentration am Beatmungsgerät gemäß Fig. 16.

Die in Fig. 17 gezeigten Kurven sind als typische Kurven-Beispiele zu verstehen. In der Realität kann es zu Abweichungen von den hier gezeigten Signalverläufen kommen. Zur Bestimmung der Öffnungs- und Schließdrücke muss der Verlauf von $f_{O_2}$ ausgewertet werden (so könnte man als Maß für die Öffnungs- und Schließdrücke z.B. die maximale Steigung von $f_{O_2}$ o.ä. verwenden).

Es ist zu beachten, dass bei niedriger $f_{O_2}$ der Diffusionswiderstand relativ erhöht ist. Ferner ist zu beachten, dass bei einer durch Druck geöffneten Lunge und niedrigem $f_{O_2}$ der nachfolgende Kollaps ein potentiell gefährlicher Vorgang ist. Da sich beim Kollaps definitionsgemäß das Lungenvolumen schnell verkleinert und im noch verbleibenden Lungenvolumen eine niedrige $f_{O_2}$ für die Sauerstoffversorgung nicht mehr ausreicht, muss die $S_{O_2}$ - Regelung schnell reagieren und $f_{O_2}$ erhöhen, um eine für den Patienten gefährliche Sauerstoffunterversorgung zu vermeiden.

Nach erfolgreicher Identifikation und erneuter Wiedereröffnung der Lunge erfolgt die anschließende Dauerbeatmung oberhalb der identifizierten Schließdrücke entsprechend zu der bereits bekannten Vorgehensweise gemäß Fig. 10.

**Patentansprüche**

1. Vorrichtung zur Bestimmung des alveolären Öffnens oder des alveolären Schließens der Lunge,
   mit einem Beatmungsgerät zum Beatmen der Lunge,
   mit einem Sättigungssensor zur Messung der Sättigung des Blutes (SO$_2$), und
   mit einer Recheneinheit, die bei Veränderung des Beatmungsdrucks aus dem resultierenden Verlauf der gemessenen Sättigung des Blutes (SO$_2$) einen Beatmungsdruck bestimmt, der dem alveolären Öffnen oder dem alveolären Schließen der Lunge entspricht.

2. Vorrichtung nach Anspruch 1, wobei ein Regelkreis vorgesehen ist, der die Sauerstoffkonzentration am Beatmungsgerät derart einstellt, dass der Ausgangswert des Sättigungssenors näherungsweise einen vorgegebenen Sättigungswert annimmt, und wobei die Recheneinheit bei Veränderung des Beatmungsdrucks aus dem resultierenden Verlauf der eingestellten Sauerstoffkonzentration einen Beatmungsdruck bestimmt, der dem alveolären Öffnen oder dem alveolären Schließen der Lunge entspricht.

3. Vorrichtung zur Bestimmung des alveolären Öffnens oder des alveolären Schließens der Lunge,
   mit einem Beatmungsgerät zum Beatmen der Lunge,
   mit einem CO$_2$-Sensor zur Messung der endtidalen CO$_2$-Konzentration im Atemstrom, und
   mit einer Recheneinheit, die bei Veränderung des Beatmungsdrucks aus dem resultierenden Verlauf der gemessenen endtidalen CO$_2$-Konzentration einen Beatmungsdruck bestimmt, der dem alveolären Öffnen oder dem alveolären Schließen der Lunge entspricht.

4. Vorrichtung zur Bestimmung des alveolären Öffnens oder des alveolären Schließens der Lunge,
   mit einem Beatmungsgerät zum Beatmen der Lunge,
   mit einem CO$_2$-Minutenvolumen-Sensor zur Messung des CO$_2$-Minutenvolumens im Atemstrom, und
   mit einer Recheneinheit, die bei Veränderung des Beatmungsdrucks aus dem resultierenden Verlauf des gemessenen CO$_2$-Minutenvolumens einen Beatmungsdruck bestimmt, der dem alveolären Öffnen oder dem alveolären Schließen der Lunge entspricht.

5. Vorrichtung zur automatischen künstlichen Beatmung der menschlichen Lunge

mit einer Steuereinheit zum automatischen Einstellen der Beatmungsdrücke am Beatmungsgerät und
mit einer Vorrichtung zur Bestimmung des alveolären Öffnens oder des alveolären Schließens der Lunge nach einem der Ansprüche 1 - 4,
wobei die seitens der Steuereinheit eingestellten Beatmungsdrücke über demjenigen Beatmungsdruck liegen, der dem alveolären Schließen der Lunge entspricht und der vorab von der Vorrichtung zur Bestimmung des alveolären Öffnens und des alveolären Schließens der Lunge bestimmt wurde.

**Claims**

1. Apparatus for determining the alveolar opening or the alveolar closing of the lung, comprising:

   an artificial ventilator for ventilating the lung,

   a saturation sensor for measuring the saturation of the blood ($SO_2$), and

   a data processor which determines during a change of the airway pressure from the resulting course of the measured saturation of the blood ($SO_2$) an airway pressure level which corresponds to alveolar opening or alveolar closing of the lung.

2. Apparatus according to claim 1, comprising a feedback control loop which controls the inspiratory oxygen fraction at the artificial ventilator such that the output of the saturation sensor is approximately equal to a given reference value, and wherein the data processor determines during a change of the airway pressure from the course of the controlled inspiratory oxygen fraction an airway pressure level which corresponds to alveolar opening or alveolar closing of the lung.

3. Apparatus for determining the alveolar opening or the alveolar closing of the lung, comprising:

   an artificial ventilator for ventilating the lung,

   a $CO_2$-sensor for measuring the endtidal $CO_2$ concentration in the breath flow, and

   a data processor which determines during a change of the airway pressure from the resulting course of the measured endtidal $CO_2$ concentration an airway pressure level which corresponds to alveolar opening or alveolar closing of the lung.

4. Apparatus for determining the alveolar opening or the alveolar closing of the lung, comprising:

   an artificial ventilator for ventilating the lung,

   a sensor to measure $CO_2$ volume exhaled per unit time, and

   a data processor which determines during a change of the airway pressure from the resulting course of the measured $CO_2$ volume exhaled per unit time an airway pressure level which corresponds to alveolar opening or alveolar closing of the lung.

5. Apparatus for automatic artificial ventilation of the human lung, comprising:

   a control unit for the automatic control of the airway pressures at an artificial ventilator,

   an apparatus for determining the alveolar opening or the alveolar closing of the lung according to one of the claims 1 - 4,

   wherein the airway pressures controlled by the control unit are above of the airway pressure, which corresponds to the alveolar closing of the lung and which was determined in advance by the apparatus for determining the alveolar opening or the alveolar closing of the lung.

**Revendications**

1. Procédé de détermination de l'ouverture alvéolaire ou de la fermeture alvéolaire du poumon, avec un appareil respiratoire pour faire respirer le poumon, avec un capteur de saturation pour mesurer la saturation du sang (SO$_2$), et avec une unité de calcul, qui, en cas de modification de la pression respiratoire, d'après l'allure résultante de la saturation mesurée du sang (SO$_2$), détermine une pression respiratoire correspondant à l'ouverture alvéolaire, ou bien à la fermeture alvéolaire du poumon.

2. Dispositif selon la revendication 1, un circuit de régulation étant prévu, réglant la concentration en oxygène sur l'appareil respiratoire, de manière que la valeur initiale du capteur de saturation prenne approximativement une valeur de saturation prédéterminée, et où l'unité de calcul en cas de modification de la pression respiratoire, à partir de l'allure résultante de la concentration en oxygène réglée, détermine une pression respiratoire qui corresponde à l'ouverture alvéolaire ou à la fermeture alvéolaire du poumon.

3. Dispositif de détermination de l'ouverture alvéolaire ou de la fermeture alvéolaire du poumon, avec un appareil respiratoire pour faire respirer le poumon, avec un capteur de CO$_2$ pour la mesure de la concentration en CO2, au stade de fin de respiration dans le flux respiratoire, et avec une unité de calcul, qui, en cas de modification de la pression respiratoire, à partir de la résultante de la concentration en CO2 mesurée, à l'état de fin de respiration, détermine une pression respiratoire correspondant à l'ouverture alvéolaire ou à la fermeture alvéolaire du poumon.

4. Dispositif de détermination de l'ouverture alvéolaire ou de la fermeture alvéolaire du poumon, avec un appareil respiratoire pour la respiration du poumon, avec un capteur de ventilation-minute de CO$_2$, pour la mesure du volume de ventilation-minute de CO$_2$ dans le flux respiratoire, et avec une unité de calcul, qui, en cas de modification de la pression respiratoire, à partir de l'allure résultante du volume de ventilation-minute de CO$_2$ mesuré, détermine une pression respiratoire qui correspond à l'ouverture alvéolaire ou à la fermeture alvéolaire du poumon.

5. Dispositif de respiration artificielle automatique du poumon humain avec une unité de commande pour le réglage automatique des pressions respiratoires sur l'appareil respiratoire, et avec un dispositif de détermination de l'ouverture alvéolaire ou de la fermeture alvéolaire du poumon, selon l'une des revendications 1 à 4, où les pressions respiratoires réglées côté unité de commande sont situées à une valeur supérieure à la pression respiratoire, correspondant à la fermeture alvéolaire du poumon et qui a été déterminée préalablement par le dispositif servant à déterminer l'ouverture alvéolaire et la fermeture alvéolaire du poumon.

Fig. 1

Druck / mbar

Insp.        Exsp.                    PIP

PEEP                          $p_{alv}$

t / s

$\dot{V}_{atem}$ / ml /s

60 / RR

t / s

$T_{insp}$      $T_{exsp}$

Fig. 2

EP 1 148 907 B1

Fig. 3

EP 1 148 907 B1

PIP →
PEEP →
RR →
I/E →
$f_{O2}$ →

Beatmungsgerät

$p_{mund}$
$\dot{V}_{atem}$

Atemmechanik

$P_{alv}$
$V_{lunge}$

Gasaustausch

$paO_2$
$paCO_2$

Fig. 4

$\dot{V}_{atem}$

$R_{ström}$

$P_{mund} = 0$

$C_{rs}$

$p_{alv}$

$p_{musk}$

Fig. 5

EP 1 148 907 B1

Fig. 6

EP 1 148 907 B1

Fig. 7

EP 1 148 907 B1

Fig. 8

Fig. 9

EP 1 148 907 B1

Fig. 10

Fig. 11

etCO$_2$, $\dot{V}_{CO2, atem}$

Beatmungsgerät

PIP, PEEP, **Soll**
I/E, RR, VT

PIP, PEEP,
**Ist** I/E, RR, VT

CO$_2$-Sensor
Fluß-Sensor

Patient

PC als Regler

Blutdruck

Überwachungsmonitor

EP 1 148 907 B1

EP 1 148 907 B1

$$\dot{V}_{CO_2, prod} = konst.$$

PIP  PEEP  RR

Gasaustausch-fläche

$$\dot{V}_{CO_2, Atem}$$

Körperwasser

$$PaCO_2 = f(V_{CO_2, speicher})$$

$$V_{CO_2, speicher}$$

Fig. 12

Fig. 13

Fig. 14

$SO_2$

$SO_2$

PIP, PEEP,  **Soll**  Beatmungsgerät

I/E, RR, VT

PIP, PEEP,

**Ist**

I/E, RR, VT

$SO_2$-Senso

Patient

PC als Regler

Blutdruck

Fig. 15  Überwachungsmonitor

EP 1 148 907 B1

Fig. 16

Fig. 17

EP 1 148 907 B1